# EUROPEAN PATENT SPECIFICATION

(11) **EP 0 863 950 B1**
(45) Date of publication and mention of the grant of the patent: **10.10.2001**
(21) Application number: 96939816.3
(22) Date of filing: 29.11.1996
(51) Int. Cl.: C09B 67/00, A61K 7/13

(54) **HAIR DYEING COMPOSITION**
HAARFÄRBEMITTEL
COMPOSITION POUR TEINDRE DES CHEVEUX

(30) Priority: 30.11.1995 DK 135695
(43) Date of publication of application: 16.09.1998
(73) Proprietor: Novozymes A/S, 2880 Bagsvaerd (DK)
(72) Inventor: AASLYNG, Dorrit, DK-2880 Bagsvärd (DK); SORENSEN, Niels, Henrik, DK-2880 Bagsvärd (DK); RORBÄK, Karen, Novo Allé DK-2880 Bagsvärd (DK)
(86) International application number: DK9600498
(87) International publication number: WO9719998

(56) References cited:
- EP-A- 0 504 005
- WO-A-94/00100
- WO-A-95/07988
- WO-A-95/33836
- WO-A-95/33837
- WO-A-96/00290
- DE-A- 4 314 317
- US-A- 3 251 742
- STN INTERNATIONAL, File CAPLUS, CAPLUS Accession No. 1991:498981, SARUNO, RINJIRO: "Hair-Dyeing Preparations Containing Melanin or Other Polyphenol Pigments and Manufacture of the Pigments"; & JP,A,03 077 813 (03-04-91).
- STN INTERNATIONAL, File CAPLUS, CAPLUS Accession No. 1995:974547, CHIVUKULA, MURALIKRISHNA et al., "Phenolic Azo Dye Oxidation by Laccase From Pyricularia Oryzae"; & APPL. ENVIRON. MICROBIOL. (1995), 61(12), 4374-77.
- F.XU et al. Biochem.Biophys.Acta 1292 (1996) 303-311

### SEQUENCE LISTING

(1) GENERAL INFORMATION:
   (i) APPLICANT:
      (A) NAME: Novo Nordisk A/S
      (B) STREET: Novo Alle
      (C) CITY: Bagsvaerd
      (D) COUNTRY: Denmark
      (E) POSTAL CODE (ZIP): DK-2880
      (F) TELEPHONE: +45 4444 8888
      (G) TELEFAX: +45 4449 3256
   (ii) TITLE OF INVENTION: An enzyme for dying hair
   (iii) NUMBER OF SEQUENCES: 2
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25 (EPO)
(2) INFORMATION FOR SEQ ID NO: 1:
   (i) SEQUENCE CHARACTERISTICS:
      (A) LENGTH: 2476 base pairs
      (B) TYPE: nucleic acid
      (C) STRANDEDNESS: double
      (D) TOPOLOGY: linear
   (ii) MOLECULE TYPE: DNA (genomic)
   (vi) ORIGINAL SOURCE:
      (A) ORGANISM: Scytalidium thermophilum
   (ix) FEATURE:
      (A) NAHE/KEY: intron
      (B) LOCATION: 349..411
   (ix) FEATURE:
      (A) NAME/KEY: intron
      (B) LOCATION: 502..559
   (ix) FEATURE:
      (A) NAME/KEY: intron
      (B) LOCATION: 632..686
   (ix) FEATURE:
      (A) NAME/KEY: intron
      (B) LOCATION: 1739..1804
   (ix) FEATURE:
      (A) NAME/KEY: CDS
      (B) LOCATION: join (106..348, 412..501, 560..631, 687..1738, 1805..2194)
   (xi) SEQUENCE DESCRIPTION: SEQ ID NO: 1:
   CTGAATTTAA ATACAGGAAG ATCGCATTCA ATCCAGCCTA GACTGCACAA TGGTTCTGCA       60
   CGACCGTCGC ACACCTGCCA ATAGTGTTAA TAACGGNCTA ATACC ATG AAG CGC TTC      117
                                                     Met Lys Arg Phe
                                                     1
   TTC ATT AAT AGC CTT CTG CTT CTC GCA GGG CTC CTC AAC TCA GGG GCC        165
   Phe Ile Asn Ser Leu Leu Leu Leu Ala Gly Leu Leu Asn Ser Gly Ala
   5                   10                  15                  20
   CTC GCG GCT CCG TCT ACA CAT CCC AGA TCA AAC CCC GAC ATA CTG CTT        213
   Leu Ala Ala Pro Ser Thr His Pro Arg Ser Asn Pro Asp Ile Leu Leu
                   25                  30                  35
   GAA AGA GAT GAC CAC TCC CTT ACG TCT CGG CAA GGT AGC TGT CAT TCT        261
   Glu Arg Asp Asp His Ser Leu Thr Ser Arg Gln Gly Ser Cys His Ser
               40                  45                  50
   CCA AGC AAC CGC GCC TGT TGG TGC TCT GGC TTC GAT ATC AAC ACG GAT        309
   Pro Ser Asn Arg Ala Cys Trp Cys Ser Gly Phe Asp Ile Asn Thr Asp
           55                  60                  65
   TAT GAG ACC AAG ACT CCA AAC ACC GGA GTG GTG CGG CGG GTTAGTATCC         358
   Tyr Glu Thr Lys Thr Pro Asn Thr Gly Val Val Arg Arg
       70                  75                  80
   CAAGTTACGT TTGACCAAGA AATGGACGTG AAGTGTGCTG ACTCTCCCGC TAG             411
   TAC ACC TTT GAT ATC ACC GAA GTC GAC AAC CGC CCC GGT CCC GAT GGG        459
   Tyr Thr Phe Asp Ile Thr Glu Val Asp Asn Arg Pro Gly Pro Asp Gly
               85                  90                  95
   GTC ATC AAG GAG AAG CTC ATG CTT ATC AAC GAC AAA CTC CTG GTAGG          506
   Val Ile Lys Glu Lys Leu Met Leu Ile Asn Asp Lys Leu Leu
           100                 105                 110
   GTCCTCTCGA ACGCCTGCGT CTGCCACACA GCGTAAAACT AACGAACCGC TAG             559
   GGC CCG ACA GTC TTC GCA AAC TGG GGC GAC ACC ATC GAG GTG ACC GTC        607
   Gly Pro Thr Val Phe Ala Asn Trp Gly Asp Thr Ile Glu Val Thr Val
               115                 120                 125
   AAC AAC CAC CTG AGA ACC AAC GGA GTAAGCGTTC GGACACAAAG CCCAGCAACC       661
   Asn Asn His Leu Arg Thr Asn Gly
           130                 135
   TAGACACACT CAACTGACCA AGTAG ACC TCC ATC CAC TGG CAC GGC TTG CAC CAA    716
                               Thr Ser Ile His Trp His Gly Leu His Gln
                                               140                 145
   AAA GGA ACC AAC TAC CAC GAC GGC GCC AAC GGC GTG ACC GAG TGT CCC        764
   Lys Gly Thr Asn Tyr His Asp Gly Ala Asn Gly Val Thr Glu Cys Pro
                   150                 155                 160
   ATC CCG CCC GGT GGC TCC CGA GTC TAC AGC TTC CGA GCG CGC CAA TAT        812
   Ile Pro Pro Gly Gly Ser Arg Val Tyr Ser Phe Arg Ala Arg Gln Tyr
               165                 170                 175
   GGA ACG TCA TGG TAC CAC TCC CAC TTC TCC GCC CAG TAT GGC AAC GGC        860
   Gly Thr Ser Trp Tyr His Ser His Phe Ser Ala Gln Tyr Gly Asn Gly
           180                 185                 190
   GTG AGC GGC GCC ATC CAG ATC AAC GGA CCC GCC TCC CTG CCC TAC GAC        908
   Val Ser Gly Ala Ile Gln Ile Asn Gly Pro Ala Ser Leu Pro Tyr Asp
       195                 200                 205
   ATC GAC CTC GGC GTC CTC CCG CTG CAG GAC TGG TAC TAC AAG TCC GCC        956
   Ile Asp Leu Gly Val Leu Pro Leu Xaa Asp Trp Tyr Tyr Lys Ser Ala
   210                 215                 220                 225
   GAC CAG CTC GTC ATC GAG ACC CTG GCC AAG GGC AAC GCT CCG TTC AGC       1004
   Asp Gln Leu Val Ile Glu Thr Leu Xaa Lys Gly Asn Ala Pro Phe Ser
                   230                 235                 240
   GAC AAC GTC CTC ATC AAC GGC ACC GCA AAG CAC CCC ACC ACT GGC GAA       1052
   Asp Asn Val Leu Ile Asn Gly Thr Ala Lys His Pro Thr Thr Gly Glu
               245                 250                 255
   GGG GAG TAC GCC ATC GTG AAG CTC ACC CCG GGC AAA CGC CAT CGC CTG       1100
   Gly Glu Tyr Ala Ile Val Lys Leu Thr Pro Asp Lys Arg His Arg Leu
               260                 265                 270
   CGG CTC ATC AAC ATG TCG GTG GAG AAC CAC TTC CAG GTC TCG CTG GCG      1148
   Arg Leu Ile Asn Met Ser Val Glu Asn His Phe Gln Val Ser Leu Ala
       275                 280                 285
   AAG CAC ACC ATG ACG GTC ATC GCG GCG GAC ATG GTC CCC GTC AAC GCC      1196
   Lys His Thr Met Thr Val Ile Ala Ala Asp Met Val Pro Val Asn Ala
   290                 295                 300                 305
   ATG ACC GTC GAC AGC CTG TTT ATG GCC GNC GGG CAG CGG TAT GAT GTT      1244
   Met Thr Val Asp Ser Leu Phe Met Ala Val Gly Gln Arg Tyr Asp Val
                   310                 315                 320
   ACC ATC GAC GCG AGC CAG GCG GTG GGG AAT TAC TGG TTC AAC ATC ACC      1292
   Thr Ile Asp Ala Ser Gln Ala Val Gly Asn Tyr Trp Phe Asn Ile Thr
               325                 330                 335
   TTT GGA GGG CAG CAG AAG TGC GGC TTC TCG CAC AAT CCG GCG CCG GCA      1340
   Phe Gly Gly Gln Gln Lys Cys Gly Phe Ser His Asn Pro Ala Pro Ala
           340                 345                 350
   GCC ATC TTT CGC TAC GAG GGC GCT CCT GAC GCT CTG CCG ACG GAT CCT      1388
   Ala Ile Phe Arg Tyr Glu Gly Ala Pro Asp Ala Leu Pro Thr Asp Pro
       355                 360                 365
   GGC GCT GCG CCA AAG GAT CAT CAG TGC CTG GAC ACT TTG GAT CTT TCA      1436
   Gly Ala Ala Pro Lys Asp His Gln Cys Leu Asp Thr Leu Asp Leu Ser
   370                 375                 380                 385
   CCG GTG GTG CAA AAG AAC GTG CCG GTT GAC GGG TTC GTC AAA GAG CCT      1484
   Pro Val Val Gln Lys Asn Val Pro Val Asp Gly Phe Val Lys Glu Pro
                   390                 395                 400
   GGC AAT ACG CTG CCG GTG ACG CTC CAT GTT GAC CAG GCC GCG GCT CCA      1532
   Gly Asn Thr Leu Pro Val Thr Leu His Val Asp Gln Ala Ala Ala Pro
               405                 410                 415
   CAC GTG TTT ACG TGG AAG ATC AAC GGG AGC GCT GCG GAC GTG GAC TGG      1580
   His Val Phe Thr Trp Lys Ile Asn Gly Ser Ala Ala Asp Val Asp Trp
           420                 425                 430
   GAC AGG CCG GTG CTG GAG TAT GTC ATG AAC AAT GAC CTG TCT AGC ATT      1628
   Asp Arg Pro Val Leu Glu Tyr Val Met Asn Asn Asp Leu Ser Ser Ile
       435                 440                 445
   CCG GTC AAG AAC AAC ATT GTG AGG GTG GAC GGA GTC AAC GAG TGG ACG      1676
   Pro Val Lys Asn Asn Ile Val Arg Val Asp Gly Val Asn Glu Trp Thr
   450                 455                 460                 465
   TAC TGG CTC GTC GAA AAC GAC CCG GAG GGC CGC CTC AGT TTG CCG CAT      1724
   Tyr Trp Leu Val Glu Asn Asp Pro Glu Gly Arg Leu Ser Leu Pro His
                   470                 475                 470
   CCG ATG CAT CTA CAC GTAAGTCACA TCCCCCACTA CCATTCGGAA TGACCACCAG       1779
   Pro Met His Leu His
               475
   GTACTGACAC CCTCCTCCTC AATAG GGA CAC GAT TTC TTT GTC CTA GGC CGC      1831
                               Gly His Asp Phe Phe Val Leu Gly Arg
                                           480                 485
   TCC CCC GAC GTC TCG CCC GAT TCA GAA ACC CGC TTC GTC TTT GAC CCG      1879
   Ser Pro Asp Val Ser Pro Asp Ser Glu Thr Arg Phe Val Phe Asp Pro
                   490                 495                 500
   GCC GTC GAC CTC CCC CGT CTG CGC GGA CAC AAC CCC GTC CGG CGC GAC      1927
   Ala Val Asp Leu Pro Arg Leu Arg Gly His Asn Pro Val Arg Arg Asp
               505                 510                 515
   GTC ACC ATG CTT CCC GCG CGC GGC TGG CTG CTG CTG GCC TTC CGC ACG        1975
   Val Thr Met Leu Pro Ala Arg Glu Trp Leu Leu Leu Ala Phe Arg Thr
           520                 525                 530
   GAC AAC CCG GGC GCG TGG TTG TTC CAC TGC CAC ATC GCG TGR CAC GTG        2023
   Asp Asn Pro Gly Ala Trp Leu Phe His Cys His Ile Ala Trp His Val
       535                 540                 545
   TCG GGC GGG TTA AGC GTC GAC TTT CTG GAG CGG CCG GAC GAG CTG CGC        2071
   Ser Gly Gly Leu Ser Val Asp Phe Leu Glu Arg Pro Asp Glu Leu Arg
   550                 555                 560                 565
   GGG CAG CTG ACG GGA GAG AGC AAG GCG GAG TTG GAG CGT GTT TGT CGC        2119
   Gly Gln Leu Thr Gly Glu Ser Lys Ala Glu Leu Glu Arg Val Cys Arg
                   570                 575                 580
   GAG TGG AAG GAT TGG GAG GCG AAG AGC CCG CAT GGG AAG ATC GAT TCG        2167
   Glu Trp Lys Asp Trp Glu Ala Lys Ser Pro His Gly Lys Ile Asp Ser
               585                 590                 595
   GGG TTG AAG CAG CGG CGA TGG GAT GCG TGAGGTAGTT GGGCGGATTG              2214
   Gly Leu Lys Gln Arg Arg Trp Asp Ala
           600                 605
   TTTAACACGT AGTGGGTAAG GTTGGGGCGG GTTTGTTTGG CGTTTTCAGG GGTTGGGGTG      2274
   CGGATGCTGG TCATCCGGGA AACGGCTCTA CAACTGGTGT CAATAGACTA ATATAGAGTG      2334
   ATCAAAGAAC TGAGGTTCTG AAAGAGGCGT GGAAGTCGCG TTGTGACTCC CTTTGCCATG      2394
   TTGGGAAGTG TGGCTCAACA TTGTGTTCAG GTTTGCTCAG GGTGATNTCG AACTGACGTN      2454
   TTGATGAGGG TTATTGCNTA GA                                               2476
(2) INFORMATION FOR SEQ ID NO: 2:
   (i) SEQUENCE CHARACTERISTICS:
      (A) LENGTH: 616 amino acids
      (B) TYPE: amino acid
      (C) STRANDEDNESS: single
      (D) TOPOLOGY: linear
   (ii) MOLECULE TYPE: protein
   (vi) ORIGINAL SOURCE:
      (A) ORGANISM: Scytalidium thermophilum
   (xi) SEQUENCE DESCRIPTION: SEQ ID NO: 2:
   Met Lys Arg Phe Phe Ile Asn Ser Leu Leu Leu Leu Ala Gly Leu Leu
   1 5 10 15
   Asn Ser Gly Ala Leu Ala Ala Pro Ser Thr His Pro Arg Ser Asn Pro
   20 25 30
   Asp Ile Leu Leu Glu Arg Asp Asp His Ser Leu Thr Ser Arg Gln Gly
   35 40 45
   Ser Cys His Ser Pro Ser Asn Arg Ala Cys Trp Cys Ser Gly Phe Asp
   50 55 60
   Ile Asn Thr Asp Tyr Glu Thr Lys Thr Pro Asn Thr Gly Val Val Arg
   65 70 75 80
   Arg Tyr Thr Phe Asp Ile Thr Glu Val Asp Asn Arg Pro Gly Pro Asp
   85 90 95
   Gly Val Ile Lys Glu Lys Leu Met Leu Ile Asn Asp Lys Leu Leu Gly
               100                 105                 110
   Pro Thr Val Phe Ala Asn Trp Gly Asp Thr Ile Glu Val Thr Val Asn
           115                 120                 125
   Asn His Leu Arg Thr Asn Gly Thr Ser Ile His Trp His Gly Leu His
       130                 135                 140
   Gln Lys Gly Thr Asn Tyr His Asp Gly Ala Asn Gly Val Thr Glu Cys
   145                 150                 155                 160
   Pro Ile Pro Pro Gly Gly Ser Arg Val Tyr Ser Phe Arg Ala Arg Gln
                   165                 170                 175
   Tyr Gly Thr Ser Trp Tyr His Ser His Phe Ser Ala Gln Tyr Gly Asn
               180                 185                 190
   Gly Val Ser Gly Ala Ile Gln Ile Asn Gly Pro Ala Ser Leu Pro Tyr
           195                 200                 205
   Asp Ile Asp Leu Gly Val Leu Pro Leu Gln Asp Trp Tyr Tyr Lys Ser
       210                 215                 220
   Ala Asp Gln Leu Val Ile Glu Thr Leu Ala Lys Gly Asn Ala Pro Phe
   225                 230                 235                 240
   Ser Asp Asn Val Leu Ile Asn Gly Thr Ala Lys His Pro Thr Thr Gly
                   245                 250                 255
   Glu Gly Glu Tyr Ala Ile Val Lys Leu Thr Pro Asp Lys Arg His Arg
               260                 265                 270
   Leu Arg Leu Ile Asn Met Ser Val Glu Asn His Phe Gln Val Ser Leu
           275                 280                 285
   Ala Lys His Thr Met Thr Val Ile Ala Ala Asp Met Val Pro Val Asn
       290                 295                 300
   Ala Met Thr Val Asp Ser Leu Phe Met Ala Xaa Gly Gln Arg Tyr Asp
   305                 310                 315                 320
   Val Thr Ile Asp Ala Ser Gln Ala Val Gly Asn Tyr Trp Phe Asn Ile
                   325                 330                 335
   Thr Phe Gly Gly Gln Gln Lys Cys Gly Phe Ser His Asn Pro Ala Pro
               340                 345                 350
   Ala Ala Ile Phe Arg Tyr Glu Gly Ala Pro Asp Ala Leu Pro Thr Asp
           355                 360                 365
   Pro Gly Ala Ala Pro Lys Asp His Gln Cys Leu Asp Thr Leu Asp Leu
       370                 375                 380
   Ser Pro Val Val Gln Lys Asn Val Pro Val Asp Gly Phe Val Lys Glu
   385                 390                 395                 400
   Pro Gly Asn Thr Leu Pro Val Thr Leu His Val Asp Gln Ala Ala Ala
                   405                 410                 415
   Pro His Val Phe Thr Trp Lys Ile Asn Gly Ser Ala Ala Asp Val Asp
               420                 425                 430
   Trp Asp Arg Pro Val Leu Glu Tyr Val Met Asn Asn Asp Leu Ser Ser
           435                 440                 445
   Ile Pro Val Lys Asn Asn Ile Val Arg Val Asp Gly Val Asn Glu Trp
       450                 455                 460
   Thr Tyr Trp Leu Val Glu Asn Asp Pro Glu Gly Arg Leu Ser Leu Pro
   465                 470                 475                 480
   His Pro Met His Leu His Gly His Asp Phe Phe Val Leu Gly Arg Ser
                   485                 490                 495
   Pro Asp Val Ser Pro Asp Ser Glu Thr Arg Phe Val Phe Asp Pro Ala
               500                 505                 510
   Val Asp Leu Pro Arg Leu Arg Gly His Asn Pro Val Arg Arg Asp Val
           515                 520                 525
   Thr Met Leu Pro Ala Arg Gly Trp Leu Leu Leu Ala Phe Arg Thr Asp
       530                 535                 540
   Asn Pro Gly Ala Trp Leu Phe His Cys His Ile Ala Trp His Val Ser
   545                 550                 555                 560
   Gly Gly Leu Ser Val Asp Phe Leu Glu Arg Pro Asp Glu Leu Arg Gly
                   565                 570                 575
   Gln Leu Thr Gly Glu Ser Lys Ala Glu Leu Glu Arg Val Cys Arg Glu
               580                 585                 590
   Trp Lys Asp Trp Glu Ala Lys Ser Pro His Gly Lys Ile Asp Ser Gly
           595                 600                 605
   Leu Lys Gln Arg Arg Trp Asp Ala
       610                 615

## Description

The present invention relates to a dyeing composition for keratinous fibres, in particular hair, fur, hide and wool, a method for dying and the use of a *Scytalidium* laccase for dyeing.

### BACKGROUND OF THE INVENTION

It has been used for many years to dye the hair to cover appearing grey hair. The need to do so arises from the fact that grey hair is the first sign of having past adolescence, which can be hard to accept for many people.

For instance, in certain parts of Asia it is widely used by both men and women to dye the hair with dyes often referred to by humorous people as "black shoe polish".

During the last few decades hair dyeing has become more and more popular in the western world. At first Punk Rockers and other society critical groups dyed their hair in extreme colours as a part of their protest against the established society, but today especially many young people also uses hair dyes (in more soft tints than the Punk Rockers) as a sort of "cosmetic" to change or freshen up their "look".

### Hair dyes

In general hair dyeing compositions on the market today can be divided into three main groups:
- temporary hair dyes,
- semi-permanent hair dyes, and
- permanent oxidative hair dyes.

The temporary hair dyes are only intended to change the natural hair colour for a short period of time and usually functions by using dyes having a high affinity for hair keratin and which is able to penetrate into the interior of the hair shaft.

Permanent hair dyes are very durable to sunlight, shampooing and other hair treatments and need only to be refreshed once a month as new hair grows out. With these dyeing systems the dyes are created directly in and on the hair. Small aromatic colourless dye precursors (*e.g.* p-phenylene-diamine and o-aminophenol) penetrate deep into the hair where said dye precursors are oxidised by an oxidising agent into coloured polymeric compounds. These coloured compounds are larger than the dye precursors and can not be washed out of the hair.

By including compounds referred to as modifiers (or couplers) in the hair dyeing composition a number of hair colour tints can be obtained. Cathecol and Resorcinol are examples of such modifiers.

Traditionally H₂O₂ is used as the oxidizing agent (colour builder), but also as a bleaching agent. Dyeing compositions comprising H₂O₂ are often referred to as "lightening dyes" due to this lightening effect of H₂O₂.

The use of H₂O₂ in dye compositions have some disadvantages as H₂O₂ damages the hair. Further, oxidative dyeing often demands high pH (normally around pH 9-10), which also inflicts damage on the hair. Consequently, if using dye compositions comprising H₂O₂ it is not recommendable to dye the hair often.

To overcome the disadvantages of using H₂O₂ it has been suggested to use oxidation enzymes to replace H₂O₂.

US patent no. 3,251,742 (Revlon) describes a method for dyeing human hair by dye formation *in situ* (*i.e.* on the hair}. An oxidative enzyme is used to the colour formation reactions at a substantially neutral pH (pH 7-8.5).

Laccases, tyrosinases, polyphenolases and catacolases are mentioned as the suitable oxidation enzymes.

EP patent no. 504.005 (Perma S.A.) concerns compositions for dying hair which do not require the presence of H₂O₂ (hydrogen peroxide). The composition comprises an enzyme capable of catalyzing the formation of the polymeric dyes and also dye precursors, such as bases and couplers, in a buffer solution wherein the pH of said composition is between 6.5 and 8 and said enzyme has an optimal activity in the pH range between 6.5 and 8.

*Rhizoctonia praticola* laccase and *Rhus vernicifera* laccase have a pH-optimum between 6.5 and 8 and can be used to form the polymeric dyes according to this patent.

Abstract of Papers American Chemical Society vol. 209, no. 1-2, 1995 discloses the cloning of a laccase from a *Scytalidium thermophilum*. The abstract does not mention the use of said laccase for dyeing hair.

### SUMMARY OF THE INVENTION

The object of the present invention is to provide improved permanent dyeing compositions for keratinous fibres, in particular hair, fur, hide and wool, which is less damaging to the keratinous fibres than *e.g.* dyeing compositions for hair using H₂O₂.

It has now surprisingly been found that it is possible to provide such an improved dyeing composition by using an enzyme derived from a strain of the filamentous fungus genus *Scytalidium* as the oxidation enzyme.

In the first aspect the invention relates to a permanent dyeing composition for keratinous fibres, in particular hair, fur, hide and wool comprising an oxidation enzyme comprising
1) one or more laccase(s) derived from a strain of the genus *Scytalidium,*
2) one or more dye precursors, and
optionally 3) one or more modifiers.

The oxidation enzyme is a laccase derived from a strain of the genus *Scytalidium*, in particular from a strain of the species *Scytalidium thermophilum*.

Secondly, it is the object of the invention to provide a method for dying keratinous fibres, comprising contacting a laccase derived from a strain of the genus Scytalidium with the keratinous fibres and at least one dye precursor in the presence or absence of at least one modifier for a suitable period of time and under conditions sufficient to permit oxidation of the dye precursor into a coloured compound.

### BRIEF DESCRIPTION OF THE DRAWING

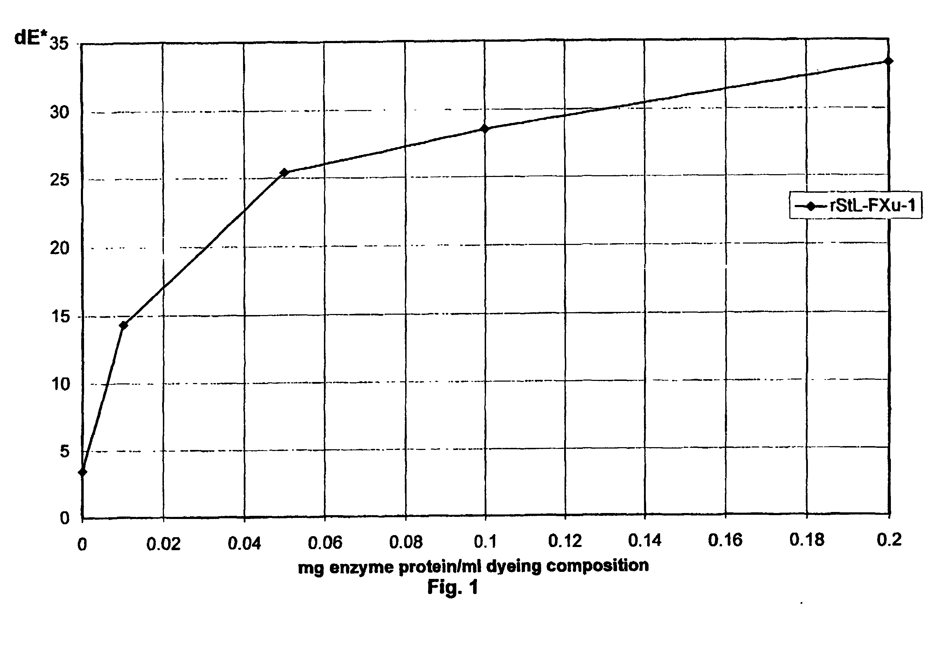

Figure 1 shows the dyeing effect of the *Scytalidium thermophilum* laccase (rStL-FXu-1)

### DETAILED DESCRIPTION OF THE INVENTION

The object of the present invention is to provide improved permanent dyeing compositions for keratinous fibres, in particular hair, fur, hide and wool, which is less damaging to the keratinous fibres than e.g. hair dyeing compositions using H₂O₂.

It has surprisingly be found that it is possible to provide such an improved dyeing composition by using an oxidation enzyme derived from a strain of the filamentous fungus genus *Scytalidium*.

When using said oxidation enzyme derived from a strain of the genus *Scytalidium* the colour developed is as wash stable as oxidative dyeing of *e.g.* hair using H₂O₂ and the light fastness is as good as when dyeing chemically.

Consequently, in the first aspect the present invention relates to a permanent dye composition for keratinous fibres , in particular hair, fur, hide and wool, comprising
1) one or more laccase(s) derived from a strain of the genus *Scytalidium*,
2) one or more dye precursors, and
optionally 3) one or more modifiers.

The oxidation enzyme is a laccase derived from a strain of genus *Scytalidium*, such as a strain of *Scytalidium thermophilum e.g.* the purified laccase described in WO 95/33837 (PCT/US95/06816) from Novo Nordisk, which is hereby incorporated. SEQ ID No 1 shows a DNA sequence encoding a suitable laccase derivable from a strain of the species *Scytalidium thermophilum.*

*E. coil* JM101 containing the expression vector pShTh15 comprising SEQ ID NO 1 has been deposited under the Budapest Treaty with the Agricultural Research Service Patent Culture Collection, Northern Regional Research Center, 1815 University Street, Peoria, Illinois, 61604. The vector have been given the Accession Number NRRL B-21262.

Also contemplated according to the invention are laccases derived from other microorganisms being more than 80% homologous to SEQ ID NO 1 derived from a strain of the species *Scytalidium thermophilum.*

In addition, *Scytalidium* laccases also encompass alternative forms of laccases which may be found in *S. thermophilum* and as well as laccases which may be found in other fungi which are synonyms of fall within the definition of *S. thermophilum* as defined by Straatsma and Samson, (1993), Mycol. Res. 97, 321-328). These include *S. indonesiacum*, *Torula thermophila*, *Humicola brevis* var. thermoidea, *Humicola brevispora, H. grisea* var. thermoidea, *Humicola insolens*, and *Humicola lanuginosa* (also known as *Thermomyces lanuginosus*).

It is to be understood that the *Scytalidium* laccase may be produced homologously, or heterologously using filamentous fungus, yeast or bacteria as the host cell.

Examples of filamentous fungi host cells include strains of the species of *Trichoderma*, preferably a strain of *Trichoderma harzianum* or *Trichoderma reesei*, or a species of *Aspergillus*, most preferably *Aspergillus oryzae* or *Aspergillus niger,* or yeast cells, such as *e.g.* a strain of *Saccharomyces*, in particular *Saccharomyces cerevisiae*, *Saccharomyces kluyveri or Saccharomyces uvarum*, a strain of *Schizosaccharomyces* sp., such as *Schizosaccharomyces pombe,* a strain of *Hansenula* sp., *Pichia* sp., *Yarrowia* sp., such as *Yarrowia lipolytica,* or *Kluyveromyces* sp., such as *Kluyveromyces lactis*, or a bacteria, such as gram-positive bacteria such as strains of *Bacillus,* such as strains of *B. subtilis*, *B. licheniformis*, *B. lentus*, *B. brevis, B. stearothermophilus*, *B. alkalophilus*, *B. amyloliquefaciens*, *B. coagulans*, *B. circulans*, *B. lautus*, *B. megaterium* or *B. thuringiensis*, or strains of *Streptomyces,* such as *S*. *lividans* or *S. murinus*, or gram-negative bacteria such as *Escherichia coli.*

Laccases (benzenediol:oxygen oxidoreductases) (E.C. class 1.10.3.2 according to Enzyme Nomenclature (1992) Academic Press, Inc) are multi-copper containing enzymes that catalyze the oxidation of phenols. Laccase-mediated oxidations result in the production of aryloxy-radical intermediates from suitable phenolic substrates; the ultimate coupling of the intermediates so produced provides a combination of dimeric, oligomeric, and polymeric reaction products. Certain reaction products can be used to form dyes suitable for dyeing hair (see below).

In an embodiment of the invention the *Scytalidium* laccase is neutral. In the context of laccases of the present invention this means that the pH optimum lies in the range from between 6.0 and 8.0.

To obtain dyeing of the keratinous fibres, such as hair, the dyeing composition of the invention also comprises a dye precursor which is converted into a coloured compound (*i.e*. a dye) by the oxidation agent which according to the invention is an oxidation enzyme derived from a strain of the species *Scytalidium*, such as a strain of *Scytalidium thermophilum.*

Without being limited thereto the dye precursor(s) may be (an) aromatic compound(s) belonging to one of three major chemical families: the diamines, aminophenols (or aminonaphtols) and the phenols. Examples of isatin derivative dye precursors can be found in DE 4,314,317-A1. Further, a number of indole or indoline derivative dye precursors are disclosed in WO 94/00100. Said dye precursors mentioned in these documents are hereby incorporated herein by reference.

Examples of such suitable dye precursors include compounds from the group comprising p-phenylene-diamine (pPD), p-toluylene-diamine, chloro-p-phenylenediamine, p-aminophenol, o-aminophenol and 3,4-diaminotoluene, 2-methyl-1,4-diaminobenzene, 4-methyl-o-phenylenediamine, 2-methoxy-p-phenylenediamine, 2-chloro-1,4-diamino-benzene, 4-amino diphenylamine, 1-amino-4-β-methoxyethylamino-benzene, 1-amino-4-bis-(β-hydroxyethyl)-amonibenzene, 1-3-diamino-benzene, 2-methyl-1,3-diamino-benzene, 2,4-diaminotoluene, 2,6-diaminopyridine, 1-hydroxy-2-amino-benzene, 1-hydroxy-3-amino-benzene, 1-methyl-2-hydroxy-4-aminobenzene, 1-methyl-2-hydroxy-4-β-hydroxyethylamino-benzene, 1-hydroxy-4-amino-ebnzene, 1-hydroxy-9-methylamino-benzene, 1-methoxy-2,4-diamino-benzene, 1-ethoxy-2,3-diamino-benzene, 1-β-hydroxyethyloxy-2,4-diamino-benzene, phenazines, such as 4,7-phenazinedicarboxylic acid, 2,7-phenazinedicarboxylic acid, 2-phenazinecarboxylic acid, 2,7-diaminophenazine, 2,8-diaminophenazine, 2,7-diamino-3,8-dimethoxyphenazine, 2,7-diamino-3-methoxyphenazine, 2,7-diamino 3-methoxyphenazine, 3-dimethyl 2,8-phenazinediamine, 2,2'-[(8-amino-7-methyl-2-phenazinyl)imino]bis-ethanol, 2,2'-[(8-amino-7-methoxy-2-phenazinyl)imino]bis-ethanol, 2,2'-[(8-amino-7-chloro-2-phenazinyl)imino]bis-ethanol, 2-[(8-amino-7-methyl-2-phenazinyl)aminol-ethanol, 2,2'-[(8-amino-2-phenazinyl)imino]bis-ethanol, 3-amino-7-(dimethylamino}-2,8-dimethyl-5-phenyl-chloride, 9-(diethylamino)-benzo[a]phenazine-1,5-diol, M-[8-(diethylamino)-2-phenazinyl]-methanesulfonamide, N-(8-methoxy-2-phenazinyl)- Methane-sulfonamide, N,N,N',N'-tetramethyl-2,7-phenazinediamine, 3,7-dimethyl-2-phenazinamine, p-amino benzoic acids, such as p-amino benzoic acid ethyl, p-amino benzoic acid glycerid, p-amino benzoic acid isobutyl, p-dimethylamino benzoic acid amil, p-dimethylamino benzoic acid octyl, p-diethoxy amino benzoic amil, p- dipropoxy amino benzoic acid ethyl, acetylsalicylic acid, isatin derivatives, such as 2,3-diamino benzoic acid.

In an embodiment the laccase is used with the dye precursor directly to oxidise it into a coloured compound. The dye precursor may be used alone or in combination with other dye precursors.

However, it is believed that when using a diamine or an aminophenol as the dye precursor at least one of the intermediate in the copolymerisation must be an ortho- or para-diamine or aminophenol. Examples of such are described in US patent no. 3,251,742 (Revlon), the contents of which are incorporated herein by reference.

Optionally the dyeing composition of the invention (especially hair dyeing compositions) also comprises a modifier (coupler) by which a number of colour tints can be obtained. In general modifiers are used in hair dyeing compositions, as the colours resulting from hair dyeing compositions without modifier(s) are usually unacceptable for most people.

Modifiers are typically m-diamines, m-aminophenols, or polyphenols. The modifier (coupler) reacts with the dye precursor(s) in the presence of the oxidative enzyme, converting it into a coloured compound.

Examples of modifiers (couplers) include m-phenylene-diamine, 2,4-diaminoanisole, 1-hydroxynaphthalene(α-naphthol), 1,4-dihydroxybenzene (hydroquinone), 1,5-dihydroxynapthalene, 1,2-dihydroxybenzene(pyrocatechol), 1,3-dihydroxybenzene (resorcinol), 1,3-dihydroxy-2-methylbenzene, 1,3-dihydroxy-4-chlorobenzene(4-chlororesorcinol), 1,2,3,trihydroxybenzene, 1,2,4-trihydroxybenzene, 1,2,4-trihydroxy-5-methylbenzene, 1,2,4-trihydroxytoluene.

In the second aspect the invention relates to a method for dying keratinous fibres, in particular hair, fur, hide and wool, comprising contacting a laccase derived from a strain of the genus *Scytalidium* with the keratinous fibres and at least one dye precursor in the presence or absence of at least one modifier, for a period of time and under conditions sufficient to permit oxidation of the dye precursor into coloured compounds (*i.e.* a dye).

The dyeing method can be conducted with one or more dye precursors, either alone or in combination with one or more modifiers.

The amount of dye precursor(s) and other ingredients used in the composition of the invention are in accordance with usual commercial amounts.

When using a *Scytalidium* laccase, such as the *Scytalidium thermophilum* laccase mentioned above, the method for dyeing keratinous fibres of the invention may be carried out at room temperature, preferably around the optimum temperature of the enzyme, at a pH in the range from 3.0 to 9.0, preferably 4.0 to 8.0, especially pH 6.0 to 8.0.

Suitable dye precursors and optional modifiers are described above.

The use of this *Scytalidlum* laccase is an improvement over the more traditional use of H₂O₂ as the latter can damage the keratinous fibres, such as hair. Further, normally prior art methods requires a high pH, which is also damaging to the keratinous fibres. In contrast hereto, the reaction with laccase can be conducted at acidic or neutral pH, and the oxygen needed for oxidation comes from the air, rather than via harsh chemical oxidation.

The result provided by the use of the *Scytalidium* laccase is comparable to that achieved with use of H₂O₂, not only in colour development, but also in wash stability and light fastness. An additional commercial advantage is that a single container package can be made containing both the laccase and the precursor, in an oxygen free atmosphere, which arrangement is not possible with the use of H₂O₂.

### MATERIALS AND METHODS

### Materials:

### Hair:

6" De Meo Virgin Natural White Hair (De Meo Brothers Inc. US)

### Enzymes:

Laccase from *Scytalidium thermophilum* described in WO 95/33837 (PCT/US95/06816) from Novo Nordisk

### Deposit of Biological Material

The following biological material has been deposited on the 25^{th} May 1994 under the terms of the Budapest Treaty with the Agricultural Research Service Patent Culture Collection, Northern Regional Research Center, 1815 University Street, Peoria, Illinois, 61604 and given the following accession number.
- Deposit: Accession Number
- *E. coli* JM101 containing pShTh15: NRRL B-21262.

### Dye precursors:

0.1 % w/w p-phenylene-diamine in 0.1 M K-phosphate buffer, pH 7.0. (pPD)
0.1 % w/w p-toluylene-diamine in 0.1 M K-phosphate buffer, pH 7.0.
0.1 % % w/w chloro-p-phenylenediamine in 0.1 M K-phosphate buffer, pH 7.0.
0.1 % w/w p-aminophenol in 0.1 M K-phosphate buffer, pH 7.0.
0.1 % w/w o-aminophenol in 0.1 M K-phosphate buffer, pH 7.0.
0.1 % w/w 3,4-diaminotoluene in 0.1 M K-phosphate, buffer pH 7.0.

### Modifiers:

0.1 % w/w m-phenylene-diamine in 0.1 M K-phosphate buffer, pH 7.0.
0.1 % w/w 2,4-diaminoanisole in 0,1 M K-phosphate buffer, pH 7.0.
0.1 % w/w a-naphthol in 0.1 M K-phosphate buffer, pH 7.0.
0.1 % w/w hydroquinone in 0.1 M K-phosphate buffer, pH 7.0.
0.1 % w/w pyrocatechol in 0.1 M K-phosphate buffer, pH 7.0.
0.1% w/w resorcinol in 0.1 M K-phosphate buffer, pH 7.0.
0.1 % w/w 4-chlororesorcinol in 0.1 M K-phosphate buffer, pH 7.0.

The dye precursor is combined with one of the above indicated modifiers so that the final concentration in the dyeing solution is 0.1 % w/w with respect to precursor and 0.1 % w/w with respect to modifier.

### Other solutions:

3% H₂O₂ (in the final dye solution)

### Commercial shampoo

### Equipment:

Minolta CR200 Chroma Meter
Day light bulb: 1000 LUX (D65)

### Determination of Laccase Activity (LACU)

Laccase activity is determined from the oxidation of syringaldazin under aerobic conditions. The violet colour produced is photometered at 530 nm. The analytical conditions are 19 mM syringaldazin, 23.2 mM acetate buffer, pH 5.5, 30°C, 1 min. reaction time.

1 laccase unit (LACU) is the amount of enzyme that catalyses the conversion of 1.0 micromole syringaldazin per minute at these conditions.

### Assessment of the hair colour

The quantitative colour of the hair tresses are determined on a Minolta CR200 Chroma Meter by the use the parameters L* ("0"=black and "100"=white), a* ("-"=green and "+"=red) and b* ("-" blue and "+" yellow).

DL*, Da* and Db* are the delta values of L*, a* and b* respectively compared to L*, a* and b* of untreated hair (e.g. DL* = L*ₛₐₘₚₗₑ - L*_{untreated hair}).

DE* is calculated as DE*=Ö(DL*²+Da*²+Db*) and is an expression for the total quantitative colour change.

### EXAMPLES

### Example 1

### Dyeing effect

The dyeing effect of a *Scytalidium thermophilum* laccase was tested using the dye precursor o-aminophenol and the modifier m-phenylenediamine.

### Hair dyeing

1 gram De Meo white hair tresses were used.

4 ml dye precursor solution (including modifier) is mixed with 1 ml laccase on a Whirley mixer, applied to the hair tresses and incubated at 30°C for 60 minutes.

The hair tresses are then rinsed with running water, washed with shampoo, rinsed with running water, combed, and air dried.

The a*, b* and L* was determined on the Chroma Meter and the DE* values were then calculated.

A hair tress sample treated without enzyme was used as a blind.

The result of the hair dyeing test is shown in figure 1.

### Example 2

### Wash stability

Tresses of white De Meo hair (1 gram) is used for testing the wash stability of hair dyed using *Scytalidium thermophilum* laccase, compared with hair dyed using H₂O₂, and p-phenylene-diamine (pPD) as the dye precursor. Further the wash stability is compared with a commercial oxidative dye.

The oxidative hair dyeing is carried out as described in Example 1.

### Hair wash

The dyed hair tresses are wetted and washed for 15 seconds with 50 ml of commercial shampoo, and rinsed with water for 1 minute and air dried. The hair tresses are washed up to 18 times.

The a*, b* and L* is determined om the Chroma Meter and the ΔE* values are then calculated.

### Example 3

### The light fastness

Tresses of blond European hair are used for testing the light fastness of hair dyed using *Scytalidium thermophilum* laccase in comparison to hair dyed using H₂O₂. p-phenylene-diamine was used as dye precursor.

The dyeing of the hair was carried out as described in Example 1.

One hair tress is kept dark, while an other is kept at day light (*i.e.* under a day light bulb (D65)), at approximately 1000 LUX) for up to 275 hours.

The a*, b* and L* parameters are determined immediately after the dyeing of the hair, and further during exposure to day light.

DE* then calculated from the determined a*, b* and L* values.

## Claims

1. A dyeing composition comprising an oxidation enzyme **characterised in that** the composition comprises:
1) one or more laccase(s) derived from a strain of the genus *Scytalidium*,
2) one or more dye precursors, and
optionally 3) one or more modifiers.

2. The dyeing composition according to claim 1, wherein the laccase is derived from a strain of the species *Scytalidium thermophilum*.

3. The dyeing composition according to claims 2 , wherein the laccase is neutral.

4. The dyeing composition according to claim 3, having the sequence shown in SEQ ID No 1.

5. The dyeing composition according to claim 4, wherein the sequence encoding the laccase is homologous to the SEQ ID NO 1.

6. The dyeing composition according to claim 5, wherein the sequence encoding the laccase is more than 80% homologous to SEQ ID NO 1.

7. The dyeing composition according to any of claims 1 to 6, comprising a dye precursor selected from the group comprising p-phenylene-diamine (pPD), p-toluylene-diamine, chloro-p-phenylenediamine, p-aminophenol, o-aminophenol and 3,4-diaminotoluene, 2-methyl-1,4-diaminobenzene, 4-methyl-o-phenylenediamine, 2-methoxy-p-phenylenediamine, 2-chloro-1,4-diamino-benzene, 4-amino diphenylamine, 1-amino-4-β-methoxyethylamino-benzene, 1-amino-4-bis-(β-hydroxyethyl)-amonibenzene, 1-3-diamino-benzene, 2-methyl-1,3-diamino-benzene, 2,4-diaminotoluene, 2,6-diaminopyridine, 1-hydroxy-2-amino-benzene, 1-hydroxy-3-aminobenzene, 1-methyl-2-hydroxy-4-amino-benzene, 1-methyl-2-hydroxy-4-β-hydroxyethylamino-benzene, 1-hydroxy-4-amino-benzene, 1-hydroxy-4-methylamino-benzene, 1-methoxy-2,4-diamino-benzene, 1-ethoxy-2,3-diamino-benzene, 1-β-hydroxyethyloxy-2,4-diamino-benzene, phenazines, such as 4,7-phenazinedicarboxylic acid, 2,7-phenazinedicarboxylic acid, 2-phenazinecarboxylic acid, 2,7-diaminophenazine, 2,8-diaminophenazine, 2,7-diamino-3,8-dimethoxyphenazine, 2,7-diamino-3-methoxyphenazine, 2,7-diamino 3-methoxyphenazine, 3-dimethyl 2,8-phenazinediamine, 2,2'-[(8-amino-7-methyl-2-phenazinyl)imino]bis-ethanol, 2,2'-[(8-amino-7-methoxy-2-phenazinyl)imino]bis-ethanol, 2,2'-[(8-amino-7-chloro-2-phenazinyl)imino]bis-ethanol, 2-[(8-amino-7-methyl-2-phenazinyl)amino]-ethanol, 2,2'-[(8-amino-2-phenazinyl)imino]bis-ethanol, 3-amino-7-(dimethylamino)-2,8-dimethyl-5-phenylchloride, 9-(diethylamino)- benzo[a]phenazine-1,5-diol, N-[8-(diethylamino)-2-phenazinyl]- methanesulfonamide, N-(8-methoxy-2-phenazinyl)- Methanesulfonamide, N,N,N',N'-tetramethyl-2,7-phenazinediamine, 3,7-dimethyl-2-phenazinamine, p-amino benzoic acids, such as p-amino benzoic acid ethyl, p-amino benzoic acid glycerid, p-amino benzoic acid isobutyl, p-dimethylamino benzoic acid amil, p-dimethylamino benzoic acid octyl, p-diethoxy amino benzoic amil, p- dipropoxy amino benzoic acid ethyl, acetylsalicylic acid, isatin derivatives, such as 2,3-diamino benzoic acid.

8. The dyeing composition according to claims 7, comprising a dye modifier selected from the group comprising m-phenylene-diamine, 2,4-diaminoanisole, 1-hydroxynaphthalene(a-naphthol), 1,4-dihydroxybenzene(hydroquinone), 1,5-dihydroxynapthalene, 1,2-dihydroxybenzene(pyrocatechol), 1,3-dihydroxybenzene (resorcinol), 1,3-dihydroxy-2-methylbenzene, 1,3-dihydroxy-4-chlorobenzene (4-chlororesorcinol), 1,2,3,trihydroxybenzene, 1,2,4-trihydroxybenzene, 1,2,4-trihydroxy-5-methylbenzene, 1,2,4-trihydroxytoluene.

9. A method for dying comprising contacting a laccase derived from a strain of the genus *Scytalidium* with the keratinous fibres and at least one dye precursor in the presence or absence of at least one modifier for a period of time and under conditions sufficient to permit oxidation of the dye precursor into a coloured compound.

10. The method according to claim 9, wherein the dyeing is carried out at a pH in the range from 3.0 to 9.0, preferably 4.0 to 8.0, especially 6.0 to 8.0.

## Patentansprüche

1. Färbezusammensetzung umfassend ein oxidatives Enzym, **dadurch gekennzeichnet, dass** die Zusammensetzung umfasst:
1) eine oder mehrere Laccase(n), die von einem Stamm der Gattung *Scytalidium* abgeleitet ist,
2) einen oder mehrere Farbstoffvorläufer und
ggf. 3) einen oder mehrere Modifikatoren.

2. Färbezusammensetzung nach Anspruch 1, in der die Laccase von einem Stamm der Spezies *Scytalidium thermophilum* abgeleitet ist.

3. Färbezusammensetzung gemäß Anspruch 2, in der die Laccase neutral ist.

4. Färbezusammensetzung gemäß Anspruch 3, die die in SEQ ID NO 1 gezeigte Sequenz aufweist.

5. Färbezusammensetzung gemäß Anspruch 4, in der die die Laccase kodierende Sequenz homolog zur SEQ ID NO 1 ist.

6. Färbezusammensetzung gemäß Anspruch 5, in der die die Laccase kodierende Sequenz zu mehr als 80% zu der SEQ ID NO 1 homolog ist.

7. Färbezusammensetzung gemäß einem der Ansprüche 1 bis 6, umfassend einen Farbstoffvorläufer, ausgewählt aus der Gruppe umfassend p-Phenylendiamin (pPD), p-Toluylendiamin, Chloro-p-phenylendiamin, p-Aminophenol, o-Aminophenol und 3,4-Diaminotoluol, 2-Methyl-1,4-diaminobenzol, 4-Methyl-o-phenylendiamin, 2-Methoxy-p-phenylendiamin, 2-Chloro-1,4-diaminobenzol, 4-Aminodiphenylamin, 1-Amino-4-β-methoxyethylaminobenzol, 1-Amino-4-bis-(ß-hydroxyethyl)-aminobenzol, 1-3-Diaminobenzol, 2-Methyl-1,3-diaminobenzol, 2,4-Diaminotoluol, 2,6-Diaminopyridin, 1-Hydroxy-2-aminobenzol, 1-Hydroxy-3-aminobenzol, 1-Methyl-2-hydroxy-4-aminobenzol, 1-Methyl-2-hydroxy-4-β-hydroxyethylaminobenzol, 1-Hydroxy-4-aminobenzol, 1-Hydroxy-4-methylaminobenzol, 1-Methoxy-2,4-diaminobenzol, 1-Ethoxy-2,3-diaminobenzol, 1-β-Hydroxyethyloxy-2,4-diaminobenzol, Phenazine, wie z. B. 4,7-Phenazindicarbonsäure, 2,7-Phenazindicarbonsäure, 2-Phenazincarbonsäure, 2,7-Diaminophenazin, 2,8-Diaminophenazin, 2,7-Diamino-3,8-dimethoxyphenazin, 2,7-Diamino-3-methoxyphenazin, 2,7-Diamino-3-methoxyphenazin, 3-Dimethyl-2,8-phenazindiamin, 2,2'-[(8-Amino-7-methyl-2-phenazinyl)imino]bis-ethanol, 2,2'-[(8-Amino-7-chloro-2-phenazinyl)imino]bis-ethanol, 2-[(8-Amino-7-methyl-2-phenazinyl)imino]bis-ethanol, 2-[(8-Amino-7-methyl-2-phenazinyl)amino]-ethanol, 2,2'-[(8-Amino-2-phenazinyl)imino]bis-ethanol, 3-Amino-7-(dimethylamino)-2,8-dimethyl-5-phenylchlorid, 9-(Diethylamino)-benzo[a]phenazin-1,5-diol, N-[8-(Diethylamino)-2-phenazinyl]-methansulfonamid, N,N,N',N'-Tetramethyl-2,7-phenazindiamin, 3,7-Dimethyl-2-phenazinamin, p-Aminobenzoesäuren, wie z. B. p-Aminobenzoesäureethyl, p-Aminobenzoesäureglycerid, p-Aminobenzoesäureisobutyl, p-Dimethylaminobenzoesäureamyl, p-Dimethylaminobenzoesäureoctyl, p-Diethoxyaminobenzoesäureamyl, p-Dipropoxyaminobenzoesäureethyl, Acetylsalicylsäure, Abkömmlinge von Isatin, wie z. B. 2,3-Diaminobenzoesäure.

8. Färbezusammensetzung gemäß Anspruch 7, umfassend einen Farbstoffmodifikator, ausgewählt aus der Gruppe umfassend m-Phenylendiamin, 2,4-Diaminoanisol, 1-Hydroxynaphthalin (α-naphthol), 1,4-Dihydroxybenzol (Hydrochinon), 1,5-Dihydroxynaphthalin, 1,2-Dihydroxybenzol (Brenzcatechin), 1,3-Dihydroxybenzol (Resorcin), 1,3-Dihydroxy-2-methylbenzol, 1,3-Dihydroxy-4-chlorbenzol (4-chlororesorcin), 1,2,3-Trihydroxybenzol, 1,2,4-Trihydroxybenzol, 1,2,4-Trihydroxy-5-methylbenzol, 1,2,4-Trihydroxytoluol.

9. Färbeverfahren, umfassend das Inkontaktbringen einer von einem Stamm der Gattung *Scytalidium* abgeleiteten Laccase mit den keratinösen Fasern und wenigstens einem Farbstoffvorläufer in der Anwesenheit oder Abwesenheit von wenigstens einem Modifikator für einen Zeitraum und unter Bedingungen, die die Oxidation des Farbstoffvorläufers in eine farbige Verbindung erlauben.

10. Verfahren gemäß Anspruch 9, in dem die Färbung bei einem pH im Bereich von 3,0 bis 9,0, vorzugsweise 4,0 bis 8,0, insbesondere 6,0 bis 8,0 ausgeführt wird.

## Revendications

1. Composition colorante comportant une enzyme d'oxydation, **caractérisée en ce que** la composition comporte :
1) une ou plusieurs laccases dérivées d'une souche du genre *Scytalldium*,
2) un ou plusieurs précurseurs de colorant, et
3) de manière facultative, un ou plusieurs modificateurs.

2. Composition colorante selon la revendication 1, dans laquelle la laccase est dérivée d'une souche de l'espèce *Scytalidium thermophilum*.

3. Composition colorante selon la revendication 2, dans laquelle la laccase est neutre.

4. Composition colorante selon la revendication 3, ayant la séquence représentée dans la SEQ ID N° 1.

5. Composition colorante selon la revendication 4, dans laquelle la séquence codant pour la laccase est homologue à la SEQ ID N° 1.

6. Composition colorante selon la revendication 5, dans laquelle la séquence codant pour la laccase a une homologie supérieure à 80 % avec la SEQ ID N° 1.

7. Composition colorante selon l'une quelconque des revendications 1 à 6, comportant un précurseur de colorant sélectionné parmi le groupe constitué des éléments suivants : p-phénylène-diamine (pPD), p-toluène-diamine, chloro-p-phénylènediamine, p-aminophénol, o-aminophénol, 3,4-diaminotoluène et 2-méthyle-1,4-diamino benzène, 4-méthyle-o-phénylène-diamine, 2-métoxy-p-phénylène-diamine, 2-chloro-1,4-diamino-benzène, 4-amino diphénylamine, 1-amino-4-β-méthoxyéthylamino-benzène, 1-amino-4-bis-(β-hydroxyéthyle)amonibenzène, 1-3-diamino-benzène, 2-méthyle-1,3-diamino-benzène, 2,4-diamino-toluène, 2,6-diaminopyridine, 1-hydroxy-2-amino-benzène, 1-hydroxy-3-aminobenzène, 1-méthyle-2-hydroxy-4-amino-benzène, 1-méthyle-2-hydroxy-4-β-hydroxy-éthylamino-benzène, 1-hydroxy-4-amino-benzène, 1-hydroxy-4-méthylamino-benzène, 1-méthoxy-2,4-diamino-benzène, 1-éthoxy-2,3-diamino-benzène, 1-β-hydroxyéthyloxy-2,4-diamino-benzène, des phénazines, tels que l'acide 4,7-phénazinedicarboxylique, l'acide 2,7-phénazinedicarboxylique, l'acide 2-phénazine-dicarboxylique, 2,7-diaminophénazine, 2,8-diaminophénazine, 2,7-diamino-3,8-diméthoxyphénazine, 2,7-diamino-3-méthoxyphénazine, 2,7-diamino-3-méthoxy phénazine, 3-diméthyle-2,8-phénazinediamine, 2,2'-[(8-amino-7-méthyle-2-phénazinyle)imino]bis-éthanol, 2,2'-[(8-amino-7-méthoxy-2-phénazinyle)imino]bis-éthanol, 2,2'-[(8-amino-7-chloro-2-phénazinyle)imino)bis-éthanol, 2-[(8-amino-7-méthyle-2-phénazinyle)amino]-éthanol, 2,2'-[(8-amino-2-phénazinyle)imino]bis-éthanol, chlorure de 3-amino-7-(diméthylamino)-2,8-diméthyle-5-phényle, 9-(di éthylamino)-benzo[a]phénazine-1,5-diol, N-[8-(diéthyl amino)-2-phénazinyle]-méthanesulfonamide, N-(8-méthoxy-2-phénazinyle)-méthanesulfonamide, N,N,N',N'-tétraméthyle-2, 7-phénazinediamine, 3,7-diméthyle-2-phénazinamine, des acides p-amino benzoïques, tels que l'éthyle d'acide p-amino benzoïque, glycéride d'acide p-amino benzoïque, isobutyle d'acide p-amino benzoïques, amile d'acide p-diméthylamino benzoïque, octyle d'acide p-diméthylamino benzoïque, amile d'acide p-diéthoxy amino benzoïque, éthyle d'acide p-dipropoxy amino benzoïque, acide acétylsalicylique, des dérivés d'isatine, tels que l'acide 2,3-diamino benzoïque.

8. Composition colorante selon la revendication 7, comportant un modificateur de colorant sélectionné parmi le groupe constitué des éléments suivants : m-phénylène-diamine, 2,4-diaminoanisole, 1-hydroxynaphtalène(α-naphtol), 1,4-dihydroxybenzène (hydroquinone), 1,5-dihydroxynaphtalène, 1,2-dihydroxybenzène(pyrocatéchol), 1,3-dihydroxybenzène (resorcinol), 1,3-dihydroxy-2-méthylbenzène, 1,3-dihydroxy-4-chloro benzène(4-chlororesorcinol), 1,2,3,trihydroxybenzène, 1,2, 4-trihydroxybenzène, 1,2,4-trihydroxy-5-méthylbenzène, 1,2,4,-trihydroxytoluène.

9. Procédé de coloration comportant la mise en contact d'une laccase dérivée d'une souche du genre *Scytalidium* avec les fibres de kératine et au moins un précurseur de colorant en présence ou l'absence d'au moins un modificateur pendant une période de temps et sous des conditions suffisantes pour permettre l'oxydation du précurseur de colorant en un composé coloré.

10. Procédé selon la revendication 9, dans lequel la coloration est effectuée à un pH situé dans la plage allant de 3,0 à 9,0, de préférence 4,0 à 8,0, en particulier de 6,0 à 8,0.
